# EUROPEAN PATENT APPLICATION

(11) **EP 2 113 560 A1**
(43) Date of publication of application: **04.11.2009**
(21) Application number: 08305133.4
(22) Date of filing: 28.04.2008
(51) Int. Cl.: C12N 5/06, A61P 37/06

(54) **Compositions for treating an arthritic condition**

(71) Applicant: TXCell, 06560 Valbonne (FR)
(72) Inventor: Foussat, Arnaud, 06410 BIOT (FR); Brun, Valérie, 06410 BIOT (FR)
(74) Representative: Pöpping, Barbara

(57) **Abstract**

The present invention relates to compositions comprising human Tr1 cells directed to a joint-associated antigen and methods for treating an arthritic condition.

## Description

### FIELD OF THE INVENTION

The present invention relates to the field of treatment of an arthritis condition. The present invention relates in particular to methods for treating an arthritis condition and diseases linked to an arthritis condition, using a medicament comprising human Tr1 cells directed against a joint-associated antigen.

### BACKGROUND

Approximately 46 millions of Americans and 100 millions of Europeans are affected by arthritis and those figures are expected to increase by the years.

Rheumatoid Arthritis (RA) is characterized as an imbalance in the immune system that causes an overproduction of pro-inflammatory cytokines, e.g. tumor necrosis factor alpha (TNFα) interleukin 1 (IL-1), and a lack of anti-inflammatory cytokines, e.g. IL-10, IL-11. RA is characterized by synovial inflammation, which progresses to cartilage destruction, bone erosion and subsequent joint deformity. The primary symptoms of RA are joint inflammation, swelling, difficulty moving, and pain. During the inflammatory process, polymorphonuclear cells, macrophages, and lymphocytes are implicated. Activated T-lymphocytes produce cytotoxins and pro-inflammatory cytokines, while macrophages stimulate the release of prostaglandins and cytotoxins. Vasoactive substances (histamine, kinins, and prostaglandins) are released at the site of inflammation and cause oedema, warmth, erythema, and pain associated with inflamed joints. In the late stage of RA, enzymes produced by the inflamed cells may digest bone and cartilage. The long-term damage results in chronic pain, loss of function, deformity, disability in the joints and even a shortened life expectancy. The prevalence of RA around world is constantly at 1.0% of total population.

Juvenile idiopathic arthritis (JIA), formerly known as juvenile rheumatoid arthritis (JRA) is the most common form of persistent arthritis in children. JIA is sometimes referred to as juvenile chronic arthritis (JCA) a term that is not precise as JIA does not encompass all forms of chronic childhood arthritis. JIA is an arthritis that causes joint inflammation and stiffness for more than 6 weeks in a child less than 16 years of age. The 3 major types of JIA are oligoarticular JIA, polyarticular JIA and systemic JIA. Oligoarticular (or pauciarticular) JIA affects 5 or fewer joints in the first 6 months of disease. Polyarticular JIA affects 5 or more joints in the first 6 months of disease. This subtype can include the affect of the neck and jaw as well as the small joints usually affected. Systemic JIA (Still's Disease) is characterized by arthritis, fever and a salmon pink rash. Systemic JIA can be challenging to diagnose because the fever and rash come and go. Systemic JIA may have internal organ involvement and lead to serositis (for example pericarditis).

Ankylosing spondylitis (AS; also known as Bechterew's disease / Bechterew syndrome / Marie Strümpell disease / Marie Struempell disease / Spondyloarthritis) is a chronic, painful, degenerative inflammatory arthritis primarily affecting spine and sacroiliac joints, causing eventual fusion of the spine; it is a member of the group of the autoimmune spondyloarthropathies with a probable genetic predisposition. Complete fusion results in a complete rigidity of the spine, a condition known as bamboo spine.

Psoriatic arthritis (also arthropathic psoriasis or psoriatic arthropathy) is a type of inflammatory arthritis that affects around 5-7% of people suffering from the chronic skin condition psoriasis. Treatment of psoriatic arthritis is similar to that of rheumatoid arthritis. More than 80% of patients with psoriatic arthritis will have psoriatic nail lesions characterised by pitting of the nails, or more extremely, loss of the nail itself (onycholysis).

Most of the current treatments of Arthritis conditions are directed to the correction of immune aberration that supposedly drives the synovial cell proliferation and cartilage erosion. Present treatment of arthritis includes first line drugs for control of pain and inflammation classified as non-steroidal anti-inflammatory drugs (NSAIDs), e.g., aspirin, ibuprofen, naproxen, etc. Secondary treatments include corticosteroids, and disease modifying anti-rheumatic drugs (DMARDs), e.g., penicillinamine, cyclophosphamide, gold salts, azathioprine, levamisole, methotrexate, leflunomide, cyclosporine, etanercept, and sulfasalazine, etc.

Controlling pain is a vital part of treating arthritis. Analgesics can only provide a temporary pain relief. They neither reduce inflammation nor slow progression of the disease. Acetaminophen (Tylenol) is the most commonly used analgesic. Narcotic analgesic drugs can also be prescribed for more severe pain.

Corticosteroids are closely related to cortisol, a hormone produced on the cortex of the adrenal glands. Treatment of rheumatoid arthritis with corticosteroids remains controversial in terms of benefit/harm trade-offs. Corticosteroids are considered as very potent drugs because of their ability to reduce swelling and inflammation rapidly. However, it is well known that corticosteroids can potentially cause serious and permanent side effects. Therefore, they may only be used in certain situations systemically or locally into a specific joint for relief, always at the lowest possible effective dose for the shortest possible duration with gradually weaning off or tapering the dose over time.

NSAIDs are distinguished from corticosteroids. NSAIDs at low doses reduce pain, and at higher doses relieve inflammation. Most NSAIDs are inhibitors of the enzyme cyclooxygenase, inhibiting non-selectively both the cyclooxygenase-1 (COX-1) and cyclooxygenase-2 (COX-2). Cyclooxygenase is the rate-limiting enzyme in catalyzing the formation of prostaglandins and thromboxane from arachidonic acid. Prostaglandins among others function as messenger molecules in the process of inflammation. COX-1 is an enzyme constitutively expressed with a "house-keeping" role in regulating many normal physiological processes. The adverse effects of NSAIDs are mainly related to their inhibition of COX-1 in kidney and gastrointestinal tracts where prostaglandins serve a protective role. COX-2 is an enzyme with low or non-detectable expression in most tissues, but can be readily induced in response to cell activation by cytokines, growth factors and tumor promoters. Therapeutic effects of NSAIDs are due to their inhibition on COX-2. While selective COX-2 inhibitors, Coxibs (celecoxib, rofecoxib, valdecoxib, parecoxib and etoricoxib), were thought to have anti-inflammatory action without disrupting gastroprotective prostaglandins, an increased cardiovascular risk was seen in clinical applications which resulted in the worldwide withdrawal of some Coxibs (rofecoxib and Valdecoxib).

While the NSAID reduces day-to-day inflammation, stronger medicines, DMARDs, are usually required for patients with persistent inflammation in several joints due to inflammatory arthritis for longer than six weeks. The DMARDs slow down the biological processes that are the driving force behind persistent inflammation. DMARDs are slow-acting anti-rheumatic drugs. The quickest-acting DMARD is methotrexate, which usually takes four to six weeks before seeing benefits. The rest of the DMARDs can take three to six months or even longer to be effective. As DMARDs suppress the immune system, serious adverse effects may occur over long-term use. Methotrexate has emerged as an effective treatment for RA either as a single agent or in combination with other DMARDs. The toxicity profile of methotrexate is well established and includes serious and sometimes fatal liver disease, pneumonitis, and cytopenias.

The most exciting progress in recent years in the treatment of RA is the development of biologic DMARDs. Elucidation of the key role of TNF-α in the pathogenesis of RA has led to the development of targeted therapeutics blocking the activity of this cytokine. In addition to anti-TNF therapy, a number of other biologic DMARDs have been developed specifically against molecules (IL-1) or cells (B cells and T cells) involved in the process of immune-related diseases. Potential advantages of biologic DMARDs over traditional DMARDs, include highly specific blockade of the target molecules critically involved in the pathogenesis, rapid onset of clinical action, minimized non-specific toxicity, long dosing intervals (every week subcutaneously or every month intravenously), possible long-term immunomodulatory effects, and improved quality of life. Biological DMARDs used for arthritis include those blocking inflammatory cytokines, specifically depleting B cells and selectively inhibiting activation of T cells.

Although a wide range of drugs are available, a successful treatment for inflammatory arthritis is still a major unmet medical need. While biologic DMARDs are offering the most promising route to slowing or even halting this disease, they work only for a proportion of patients: even for the most effective anti-TNF therapy, at least one third of RA patients do not respond.

In the present invention, the Applicant aim to provide an alternative treatment for arthritis based on the use of Tr1 cells directed to a joint-associated antigen.

### SUMMARY OF THE INVENTION

The invention relates to a composition comprising at least one human Tr1 cell population directed against a joint-associated antigen, provided said joint-associated antigen is not HSP, keratin, pyruvate deshydrogenase, topoisomerase I, cardiolipin or collagen type IV.

In a preferred embodiment, said human Tr1 cell population is a human Tr1 clone population.

In an embodiment of the invention, said joint-associated antigen is selected from the group comprising citrulline-substituted cyclic and linear filaggrin peptides, collagen type II peptides, human cartilage glycoprotein 39 (HCgp39) peptides, heterogenous nuclear ribonucleoprotein (hnRNP) A2 peptides, hnRNP B1, hnRNP D, Ro60/52, BiP, vimentin, fibrinogen, collagen type I, III and V peptides, annexin V, Glucose 6 phosphate isomerase (GPI), acetyl-calpastatin, aldolase, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

In a preferred embodiment, said joint-associated antigen is collagen type II and fragments, variants and mixtures thereof. In another preferred embodiment, said joint-associated antigen is HCgp39 and fragments, variants and mixtures thereof.

Another object of the invention is a medicament comprising at least one human Tr1 cell population directed against a joint-associated antigen.

Another object of the invention is a pharmaceutical composition comprising at least one human Tr1 cell population directed against a joint-associated antigen in combination with one or more pharmaceutically acceptable carrier.

In a preferred embodiment, said human Tr1 cell population is a human Tr1 clone population.

In an embodiment of the invention, said medicament or pharmaceutical composition comprises at least one human Tr1 cell directed against a joint-associated antigen selected among citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

In a preferred embodiment, said human Tr1 cell is directed against a joint-associated antigen selected among collagen type II, HCgp39 and HSP.

The invention relates to the use of a composition comprising at least one human Tr1 cell population directed against a joint-associated antigen for the preparation of a medicament or a pharmaceutical composition for treating an arthritic condition.

In one embodiment of the invention, said joint-associated antigen is selected from the group comprising citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

In a preferred embodiment, said at least one human Tr1 cell population is directed against a joint-associated antigen selected among collagen type II, HCgp39 and HSP.

In a preferred embodiment, said arthritic condition is rheumatoid arthritis.

In another preferred embodiment, said arthritic condition is ankylosing spondylitis.

In another preferred embodiment, said arthritic condition is juvenile idiopathic arthritis.

In another preferred embodiment, said arthritic condition is psoriatic arthritis.

In one embodiment of the invention, the medicament or pharmaceutical composition is to be administered to a subject in need thereof comprises human Tr1 cells autologous to the cells of said subject.

In a preferred embodiment, 10⁴/kg to 10⁹/kg Tr1 cells are administered to the subject in need thereof.

In one embodiment of the invention, the administration to said subject of an effective amount of the medicament or the pharmaceutical composition of the invention is in combination with one or more therapeutic agent used for treating an arthritic condition.

In a preferred embodiment, said one or more therapeutic agent is selected in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, cyclosporine, minocycline, D-penicillamine.

In another embodiment of the invention, the medicament or the pharmaceutical composition of the invention is to be administrated to a subject that does not respond adequately to, or is unlikely to respond adequately to, one or more therapeutic agent in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

Another object of the invention is a process for treating an arthritic condition in a subject in need thereof, said process comprising the steps of:
- obtaining Tr1 cells directed to a selected joint-associated antigen, said Tr1 cells being obtained from a blood sample of said subject,
- cloning said Tr1 cells directed to a selected joint-associated antigen,
- further expanding Tr1 clones obtained at the previous step,
- re-injecting Tr1 clones thus obtained in said subject, preferably by intravenous route.

### BRIEF DESCRIPTION OF THE DRAWINGS

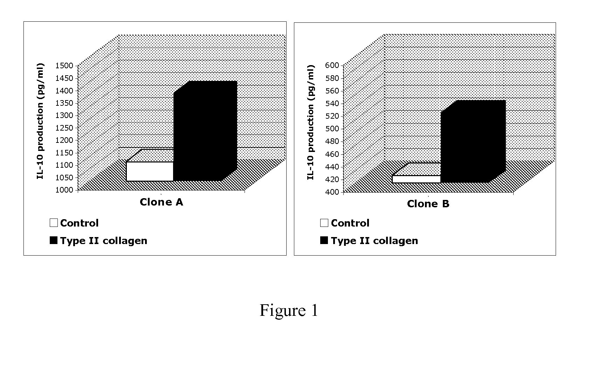

**Figure 1**: IL-10 production of Tr1 clones after specific activation with Type II collagen.
   Figure 1 describes the specific increase of the IL-10 production by T-cell clones in the presence of the specific antigen type II collagen. Clones were activated with or without type II collagen in the presence of irradiated autologous antigen presenting cells. After 48 hours, the IL-10 production was measured by ELISA.
**Figure 2****:** Cytokine secretion profile of type II collagen Tr1 clones
   IL-10, IL-4 and IFNγ secretion of type II collagen specific Tr1 cell clones were measured in 48 hours supernatant of anti-CD3 + anti-CD28 monoclonal antibodies activated cells.
**Figure 3****:** In vitro suppressive activity of type II collagen Tr1 clones
   The suppressive activity of collagen type II Tr1 clones was evaluated in coculture experiments with autologous CD4+ T lymphocytes. Cell populations were co-cultured during 3 days using anti-CD3 + anti-CD28 monoclonal antibodies. Then, cell proliferation of the autologous CD4+ T cells was assessed in the absence or presence of graded quantities of Tr1 cells. Results show that the addition of Tr1 cells to CD4+ T lymphocytes massively inhibits T-cell proliferation.

### DETAILED DESCRIPTION OF THE INVENTION

### Definition

The term "Tr1 cells" as used herein refers to cells having the following phenotype at rest CD4+CD25-FoxP3- and capable of secreting high levels of IL-10 and significant levels TGF-β upon activation. Tr1 cells are characterized, in part, by their unique cytokine profile: they produce high levels of IL-10, significant levels of TGF-β and intermediate levels of IFN-γ, but little or no IL-4 or IL-2. The cytokine production is typically evaluated in cultures of cells after activation with polyclonal activators of T lymphocytes such as anti-CD3+ anti-CD28 antibodies or Interleukin-2, PMA + ionomycin, Alternatively, the cytokine production is evaluated in cultures of cells after activation with the specific T-cell antigen presented by antigen presenting cells. High levels of IL-10 correspond to at least about 500 pg/ml, typically greater than about 1, 2, 4, 6, 8, 10, 12, 14, 16, 18, or 20 thousand pg/ml or more. Significant levels of TGF-β correspond to at least about 100 pg/ml, typically greater than about 200, 300, 400, 600, 800, or 1000 pg/ml or more. Intermediate levels of IFN-γ correspond to concentrations comprised between 0 pg/ml and at least 400 pg/ml, typically greater than about 600, 800, 1000, 1200, 1400, 1600, 1800, or 2000 pg/ml or more. Little or no IL-4 or IL-2 corresponds to less than about 500 pg/ml, preferably less than about 250, 100, 75, or 50 pg/ml, or less.

The term "antigen" as used herein refers to a protein, or peptide for which the cells of this invention are being used to modulate, or for use in any of the methods of this invention. In one embodiment, the term "antigen" may refer to a synthetically derived molecule, or a naturally derived molecule, which shares sequence homology with an antigen of interest, or structural homology with an antigen of interest, or a combination thereof. In one embodiment, the antigen may be a mimetope. A "fragment" of the antigen refers to any subset of the antigen, as a shorter peptide. A "variant" of the antigen refers to a molecule substantially similar to either the entire antigen or a fragment thereof. Variant antigens may be conveniently prepared by direct chemical synthesis of the variant peptide, using methods well-known in the art.

The term "subject" as used herein refers to a human being.

The term "effective amount" as used herein refers to an amount sufficient to cause a beneficial or desired clinical result (e.g. improvement in clinical condition).

The term "clone" or "clone population" as used herein refers to a population of differentiated cells being derived from a unique differentiated cell.

The term "treatment" or "treating" as used herein generally refers to a clinical intervention in an attempt to alter the natural course of the individual being treated, and may be performed during the course of clinical pathology. Desirable effects include, but are not limited to, alleviating symptoms, suppressing, diminishing or inhibiting any direct or indirect pathological consequences of the disease, lowering the rate of disease progression, ameliorating or palliating the disease state, and causing remission or improved prognosis. In the context of the invention, it refers to any improvement in the clinical symptoms of the inflammatory arthritis, as well as any improvement in the well being of the patients, in particular an improvement manifested by at least one of the following: decreased swelling and tenderness of the joints, decrease in pain in the joints, improved motility, slowing of the deterioration of the joints and the surrounding tissue, increase in the remission period between acute disease attacks; decrease in the time length of the acute attack; prevention of the onset of severe disease, etc.

The term "arthritis" as used herein refers to chronic inflammation, (regardless of the cause but typically due to an autoimmune process that affects the joints), in the tissue around the joints, such as the tendons, ligaments, and muscles, as well as other organs in the body. Preferably the inflammatory arthritis being treated is rheumatoid arthritis (RA), juvenile idiopathic arthritis (JIA), ankylosing spondylitis, psoriatic arthritis, polychondritis, septic arthritis. A preferred therapy target is rheumatoid arthritis.

### The present invention

The present invention relates to a method for treating an arthritis condition in a subject in need thereof, comprising the administration to said subject of a composition comprising human Tr1 cells directed against a joint-associated antigen.

According to the invention, the "human Tr1 cell population" corresponds to Tr1 cells as described here above in the definitions and does not include CD4+CD25+ regulatory T cells or FoxP3+ regulatory T cells (natural or conventional Treg), TGF-β secreting Th3 cells, or regulatory NKT cells.

According to the invention, the term "joint-associated antigen" refers to an immunogenic peptide, which is present in the joint.

In one embodiment of the invention, said immunogenic peptide may be present in the resting joint.

In another embodiment, said immunogenic peptide may be present in an inflammatory joint.

Examples of joint-associated antigens include, but are not limited to, citrulline-substituted cyclic and linear filaggrin peptides, collagen type II peptides, human cartilage glycoprotein 39 (HCgp39) peptides, heterogenous nuclear ribonucleoprotein (hnRNP) A2 peptides, hnRNP B1, hnRNP D, Ro60/52, HSP60, 65, 70 and 90, BiP, keratin, vimentin, fibrinogen, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic cardiolipin and phosphatidylserine, neutrally charged phosphatidylethanolamine and phosphatidylcholine, matrix metalloproteinase, fibrillin, aggreccan.

In a preferred embodiment, said human Tr1 cells are directed against a joint-associated antigen selected among collagen type II, HCgp39 and HSP proteins.

In a more preferred embodiment, said composition comprises human Tr1 cells directed against type II collagen. Human Tr1 cells directed against type II collagen may be directed against epitopes present in the 245-273 fragment of type II collagen (IAGAPGFPGPRGPPGPQGATGPLGPKGQT, SEQ ID NO 1) and associated either with HLA-DR1 or HLA-DR4 subjects.

In another more preferred embodiment, said composition comprises human Tr1 cells directed against HCgp39. Human Tr1 cells directed against HCgp39 may be directed against the following epitopes of HCgp39: PTFGRSFTLASSE (SEQ ID NO 2), RSFTLASSETGVG (SEQ ID NO 3), VGYDDQESVKSKV (SEQ ID NO 4), SQRFSKIASNTQSR (SEQ ID NO 5), FGRSFTLAS (SEQ ID NO 6), FTLASSETG (SEQ ID NO 7), YDDQESVKS (SEQ ID NO 8), FSKIASNTQ (SEQ ID NO 9).

In another more preferred embodiment, said composition comprises human Tr1 cells directed against HSP proteins, such as HSP60, HSP70, HSP90.

Without whishing to be bound to a theory, the Applicant assume that the injected Tr1 cell population directed to a joint-associated antigen would be activated in vivo by the antigen present in the joint and then would be able to control an arthritic condition.

The present invention relates to a composition comprising at least one human Tr1 cell population directed against a joint-associated antigen, provided that said human Tr1 cell population is not directed against HSP, collagen type IV, keratin, pyruvate deshydrogenase, topoisomerase I, and cardiolipin.

In a preferred embodiment, said composition comprises a human Tr1 cell population directed against joint-associated antigen selected among collagen type II and HCgp39.

In a more preferred embodiment, said composition comprises a human Tr1 cell population directed against collagen type II.

In another more preferred embodiment, said composition comprises a human Tr1 cell population directed against HCgp39.

In one embodiment of the invention, human Tr1 cells may be obtained by
a) isolating a progenitor cell population from a subject,
b) obtaining a population of dendritic cells by culturing said progenitor cell population in the presence of IL-10,
c) contacting cells of step b) with a CD4+ T lymphocyte population isolated from said subject in the presence of a joint-associated antigen to allow differentiation of CD4+ T cells directed to said antigen into the Tr1 cell population, and
d) recovering the Tr1 cell population from the step c).

In step b), IL-10 is present from 50 to 250 U/ml, preferably at 100 U/ml in the culture medium. Said method for obtaining Tr1 cells is described in Wakkach et al (Immunity 2003 May; 18(5):605-17).

Said method may also be carried out using Dexamethasone and Vitamin D3, or tolerogenised or immature DCs instead of the DCs of step b).

In another embodiment of the present invention, human Tr1 cells may be obtained by:
a) culturing a CD4+ T cell population directed to a joint-associated antigen isolated from a subject in a media with an appropriate amount of IFN-α, and
b) recovering the Tr1 cell population.

IFN-α is preferably present in the media at 5 ng/ml. In the step a), the media may further comprise an appropriate amount of IL-10, preferably at 100 U/ml.

In step b), the Tr1 cell population is cultured in a media comprising IL-15 to allow proliferation, IL-15 being preferably at 5 ng/ml in the media. Said method for obtaining Tr1 cells is described in the patent US6746670.

In still another embodiment of the invention, human Tr1 cells may be obtained by:
a) in vitro activating a CD4+ T cell population in presence of a joint-associated antigen, presented by artificial antigen presenting cells, and
b) recovering an activated CD4+ T cells comprising at least 10% of Tr1 cells.

Preferably, the artifical antigen presenting cells express a HLA II system molecule and a human LFA-3 molecule and don't express the co-stimulation molecules B7-1, B7-2, B7-H1, CD40, CD23 and CAM-1.

Said process, for obtaining Tr1 cells is described in the patent application WO02/092793.

In still another embodiment of the invention, human Tr1 cells may be obtained by:
a) in vitro activating a CD4+ T cell population in presence of a joint-associated antigen and an appropriate amount of IL-10; and
b) recovering the Tr1 cell population.

Preferably, IL-10 is present in the media at 100 U/ml. Said method is described in Groux et al. (Nature 1997, 389(6652):737-42).

In still another embodiment of the invention, human Tr1 cells may be obtained by:
a) stimulating a leukocyte population or a peripheral blood mononuclear cell (PBMC) population with a joint-associated antigen,
b) recovering the antigen-specific Tr1 cell population from the stimulated population,
c) optionally expanding said antigen-specific Tr1 cell population.

Leukocytes encompass several types of cells, which are characterized by their importance, their distribution, their number, their lifetime and their potentiality. These types are the following : the polynuclear or granular leukocytes, among which one finds the eosinophilic, the neutrophilic and the basophilic leukocytes, and the mononuclear cells, or peripheral blood mononuclear cells (PBMCs), which are large white blood cells and consist in the cell types of the immune system (lymphocytes and monocytes). The leukocytes or the PBMCs can be separated from the peripheral blood by any method known to those skilled in the art. Advantageously, for the separation of the PBMCs, centrifugation may be used, preferably density gradient centrifugation, preferably discontinuous density gradient centrifugation. An alternative is the use of specific monoclonal antibodies. In certain embodiments PBMC are typically isolated from the whole blood product by means of Ficoll-Hypaque, using standard procedures. In other embodiments the PBMCs are recovered by means of leukapheresis.

Said method is described in the patent application WO2007/010406.

In still another embodiment, human Tr1 cells may be obtained by:
a) culturing a leukocyte population or a peripheral blood mononuclear cell (PBMC) population with mesenchymal stem cells in the presence of a joint-associated antigen,
b) recovering the Tr1 cell population.

Said method can also be carried out with naive or memory T cells instead of PBMC or leukocytes.

The Tr1 cell population thus obtained may further be expanded by culture in presence of cytokines such as Interleukin-2 and Interleukin-4. Alternatively, Interleukin-15 and Interleukin-13 could also be used in Tr1 cell expansion cultures.

In the methods described above, human Tr1 cells can be characterized by the identification method described in WO2005/000344. Said identification method of Tr1 cells is based on the detection of the simultaneous presence of expression products of genes coding CD4 molecule and molecules from the group comprising CD18 and/or CD11a, and CD49b. Tr1 cells can be identified and/or purified by Elisa, flow cytometry, or immunoaffinity methods with antibodies directed against said markers.

Tr1 cells can also be enriched by positive selection or negative selection using flow cytometry or magnetic beads. Such methods are also described in WO2005/000344.

In another embodiment of the present invention, the Tr1 cells directed to a joint-associated antigen may be expanded by the in vitro method described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In a preferred embodiment of the present invention, said Tr1 cells directed to joint-associated antigen may be cloned by using conventional methods for cloning T cells.

In preferred embodiment of the present invention, said composition comprising at least one human Tr1 cell population directed against a joint-associated antigen or at least one clone of human Tr1 cell directed against a joint-associated antigen may be frozen to be stored.

In a preferred embodiment of the present invention, said joint-associated antigen is selected from the group comprising citrulline-substituted cyclic and linear filaggrin peptides, collagen type II peptides, human cartilage glycoprotein 39 (HCgp39) peptides, heterogenous nuclear ribonucleoprotein (hnRNP) A2 peptides, hnRNP B1, hnRNP D, Ro60/52, BiP, vimentin, fibrinogen, collagen type I, III, and V peptides, annexin V, Glucose 6 phosphate isomerase (GPI), acetyl-calpastatin, aldolase, snRNP, PARP, Scl-70, Scl-100, neutrally charged phosphatidylethanolamine and phosphatidylcholine, matrix metalloproteinase, fibrillin, aggreccan, fragments, variants and mixtures thereof. Preferably, the joint-associated antigen is a recombinant or a synthesized antigen.

Preferably, said joint-associated antigen is collagen type II, fragments, variants and mixtures thereof.

Preferably, said joint-associated antigen is HCgp39, fragments, variants and mixtures thereof.

The term "variant" of the joint-associated antigen refers herein to an antigen that is almost identical to the natural antigen and which shares the same biological activity. The minimal difference between the natural antigen and its variant may lie for example in an amino-acid substitution, deletion, and/or addition. Such variants may contain for example conservative amino acid substitutions in which amino acid residues are replaced with amino acid residues having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Another object of the present invention is to provide a medicament comprising at least one human Tr1 cell population directed against a joint-associated antigen.

The present invention also intends to provide a pharmaceutical composition comprising at least one human Tr1 cell population directed against a joint-associated antigen in combination with one or more pharmaceutically acceptable carrier.

According to a preferred embodiment, said human Tr1 cell population is a human Tr1 clone population.

According to a preferred embodiment, said joint-associated antigen is selected among citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

According to a more preferred embodiment, the medicament or the pharmaceutical composition of the invention comprises at least one human Tr1 cell population or clone directed against a joint-associated antigen selected among collagen type II, HCgp39 and HSP.

Preferably, said composition comprises human Tr1 cells directed against type II collagen. Human Tr1 cells directed against type II collagen may be directed against epitopes present in the 245-273 fragment of type II collagen (IAGAPGFPGPRGPPGPQGATGPLGPKGQT, SEQ ID NO 1) and associated either with HLA-DR1 or HLA-DR4 subjects.

In another more preferred embodiment, said composition comprises human Tr1 cells directed against HCgp39. Human Tr1 cells directed against HCgp39 may be directed against the following epitopes of HCgp39: PTFGRSFTLASSE (SEQ ID NO 2), RSFTLASSETGVG (SEQ ID NO 3), VGYDDQESVKSKV (SEQ ID NO 4), SQRFSKIASNTQSR (SEQ ID NO 5), FGRSFTLAS (SEQ ID NO 6), FTLASSETG (SEQ ID NO 7), YDDQESVKS (SEQ ID NO 8), FSKIASNTQ (SEQ ID NO 9).

In another more preferred embodiment, said composition comprises human Tr1 cells directed against HSP proteins, such as HSP60, HSP65, HSP70, HSP90.

The pharmaceutically acceptable carriers useful herein are conventional. Remington's Pharmaceutical Sciences 16^{th} edition, Osol, A. Ed. (1980) describes composition and formulations suitable for pharmaceutical delivery of the composition of the present invention. In general, the nature of the carrier will depend on the mode of administration being employed. For instance, parenteral formulations usually comprise injectable fluids that include pharmaceutically and physiologically acceptable fluids such as water, physiological saline, balanced salt solutions, aqueous dextrose, sesame oil, glycerol, ethanol, combinations thereof, or the like, as vehicle. The carrier and composition can be sterile, and the formulation suits the mode of administration. In addition to biological neutral carriers, pharmaceutical compositions to be administrated can contain minor amounts of non toxic auxiliary substances, such as wetting or emulsifying agents, preservatives, and pH buffering agents and the like, for example sodium acetate or sorbitan monolaurate. The composition can be a liquid solution, suspension, emulsion.

The present invention relates to the use of a composition comprising at least one human Tr1 cell population directed against a joint-associated antigen for the preparation of a medicament or a pharmaceutical composition for treating an arthritic condition.

Said arthritic condition includes, but is not limited to, rheumatoid arthritis, polychondritis, septic arthritis, spondyloarthropathies or ankylosing spondylitis, juvenile idiopathic arthritis (JIA), psoriatic arthritis and diseases associated with arthritis such as systemic lupus erythematous, Sjögren's syndrome, scleroderma, dermatomyosotis, polymyosotis, polymyalgia rheumatica, fibromyalgia, sarcoidosis, vasculitis.

According to a preferred embodiment, said human Tr1 cell population is a human Tr1 clone population.

According to a preferred embodiment, said one human Tr1 cell population or clone is directed against a joint-associated antigen selected among citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

According to a more preferred embodiment, said at least one human Tr1 cell population or clone is directed against a joint-associated antigen selected among collagen type II, HCgp39 and HSP.

Preferably, human Tr1 cells or clones are directed against type II collagen. Human Tr1 cells or clones directed against type II collagen may be directed against epitopes present in the 245-273 fragment of type II collagen (IAGAPGFPGPRGPPGPQGATGPLGPKGQT, SEQ ID NO 1) and associated either with HLA-DR1 or HLA-DR4 subjects.

In another more preferred embodiment, human Tr1 cells or clones are directed against HCgp39. Human Tr1 cells or clones directed against HCgp39 may be directed against the following epitopes of HCgp39: PTFGRSFTLASSE (SEQ ID NO 2), RSFTLASSETGVG (SEQ ID NO 3), VGYDDQESVKSKV (SEQ ID NO 4), SQRFSKIASNTQSR (SEQ ID NO 5), FGRSFTLAS (SEQ ID NO 6), FTLASSETG (SEQ ID NO 7), YDDQESVKS (SEQ ID NO 8), FSKIASNTQ (SEQ ID NO 9).

In another more preferred embodiment, human Tr1 cells or clones are directed against HSP proteins, such as HSP60, HSP65, HSP70, HSP90.

In a preferred embodiment, the present invention relates to the use of a composition as described here above for the preparation of a medicament or a pharmaceutical composition for treating rheumatoid arthritis.

In another preferred embodiment, the present invention relates to the use of a composition as described here above for the preparation of a medicament or a pharmaceutical composition for treating psoriatic arthritis.

In another preferred embodiment, the present invention relates to the use of a composition as described here above for the preparation of a medicament or a pharmaceutical composition for treating ankylosing spondylitis.

In another preferred embodiment, the present invention relates to the use of a composition as described here above for the preparation of a medicament or a pharmaceutical composition for treating juvenile idiopathic arthritis.

An object of the present invention is also a method for treating an arthritic condition, preferably rheumatoid arthritis, juvenile idiopathic arthritis, psoriatic arthritis or ankylosing spondylitis in a subject in need thereof, comprising administering to said subject an effective amount of a medicament as described here above or a pharmaceutical composition as described here above.

The composition may be formulated for parenteral, intramuscular, intravenous, intraperitoneal, injection, intranasal inhalation, lung inhalation, intradermal, intra-articular, intrathecal. In one embodiment of the invention, the medicament or pharmaceutical composition of the invention may be injected within the joint to be treated.

Preferably, the medicament or pharmaceutical composition of the invention may be administrated by intra-articular, intraperitoneal or intravenous injection, or by direct injection into the lymph nodes of the patient, preferably by intravenous injection.

The amount of Tr1 cells directed to a joint-associated antigen effective in the treatment of an arthritic condition will depend on the nature of the inflammation, and can be determined by standard clinical techniques. The precise dose to be employed in the formulation will also depend on the route of administration, and the seriousness of the disease or disorder, and should be decided according to the judgment of the practitioner and each individual's circumstances. Effective doses can be extrapolated from dose-response curves derived from in vitro or animal model test systems.

In one embodiment of the present invention, 10⁴/kg to 10⁹/kg cells are administrated to the subject. Preferably 10⁵/kg to 10⁷/kg cells and more preferably about 10⁶/kg cells are administrated to the subj ect.

In one embodiment of the invention, the subject is administrated with the medicament at the time when flare-up are demonstrated by a decline in the clinical status of the subject or at the time when inflammatory lesions can be visualized for example by radiography or magnetic resonance imaging.

In one embodiment of the invention, the subject is administrated once with the medicament or the pharmaceutical composition of the present invention.

In a second embodiment of the invention, the subject is administrated once a month with the medicament or the pharmaceutical composition of the present invention.

In a third embodiment of the invention, the subject is administrated once a quarter with the medicament or the pharmaceutical composition of the present invention.

In a fourth embodiment of the invention, the subject is administrated once to twice a year with the medicament or the pharmaceutical composition of the present invention.

In another embodiment of the present invention, the medicament or pharmaceutical composition to be administered to a subject in need thereof comprises human Tr1 cells autologous to the cells of said subject.

This means that Tr1 cells will be administrated to the subject they come from or that precursors used for the production of Tr1 cells come from the subject the Tr1 cells will be administrated to.

The present invention relates also to a process for treating an arthritic condition in a subject in need thereof, said process comprising the steps of:
- collecting a blood sample of said subject,
- obtaining Tr1 cells directed to a selected joint-associated antigen,
- cloning said Tr1 cells directed to a selected joint-associated antigen,
- further expanding Tr1 clones obtained at the previous step,
- injecting Tr1 clones thus obtained in said subject, preferably by intravenous route.

Preferably, cloning and expansion of Tr1 clones directed to a selected joint-associated antigen is carried out with the following method:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In another embodiment of the present invention, the method for treating an arthritic condition in a subject in need thereof comprises the administration to said subject of an effective amount of the medicament or the pharmaceutical composition of the invention in combination with one or more therapeutic agent used for treating an arthritic condition.

The present invention relates to the use of the pharmaceutical composition or medicament of the invention, wherein the administration to said subject of an effective amount of the medicament or the pharmaceutical composition of the invention is in combination with one or more therapeutic agent used for treating an arthritic condition.

Examples of therapeutic agents commonly used for treating an arthritic condition are the following:
- corticoids (prednisone),
- anti-TNF such as Infliximab, Adalimumab, Etanercept;
- anti-interleukins such as Anakinra, AMG108, Iguratimod, Actemra
- anti-B lymphocytes such as Rituximab, Epratuzumab;
- anti- costimulatory molecules such as Abatacept, Belimumab;
- tolerogenic agents (synthetic molecules directed to B lymphocyte surface DNA receptors) such as LJP 394 or TV-4710;
- anti-complement protein such as Eculizumab;
- Inhibitors of T cell signalling molecules such as CP690550
- Inhibitors of cell migration such as antagonist of chemokine receptors (Maraviroc, INCB3284)
- leflunomide,
- sulfasalazine,
- hydroxychloroquine,
- azathioprine,
- methotrexate
- cyclosporine,
- minocycline,
- D-penicillamine,
- combination therapy thereof such as methotrexate + sulfasaline, methothrexate + hydroxychloroquine, methotrexate + azathioprine, methotrexate + infliximab, methotrexate + leflunomide, methotrexate + etanercept, cyclosporine + hydroxychloroquine, cyclosporine + methotrexate, methotrexate + sulfasalazine + hydroxychloroquine.

In a preferred embodiment of the present invention, the method for treating an arthritic condition in a subject in need thereof comprises the administration to said subject of an effective amount of the medicament or the pharmaceutical composition of the invention in combination with one or more therapeutic agent selected in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

The present invention relates to the use of the pharmaceutical composition or medicament of the invention for treating an arthritic condition in a subject in need thereof, wherein the administration to said subject of an effective amount of the medicament or the pharmaceutical composition of the invention is in combination with one or more therapeutic agent selected in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

In another embodiment, the present invention also relates to a method of treatment of an arthritic condition in which the medicament or the pharmaceutical composition of the invention is to be administrated to a subject in need thereof, wherein the subject does not respond adequately to, or is unlikely to respond adequately to, one or more therapeutic agent in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

The present invention relates to the use of the pharmaceutical composition or medicament of the invention, wherein said subject does not respond adequately to, or is unlikely to respond adequately to, one or more therapeutic agent in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine

"Inadequate response", "does not respond adequately to", or "unlikely to respond adequately" refer to an actual or probable response by a subject which indicates that the therapy has been, or is likely to be, ineffective, toxic, or poorly tolerated insofar as the subj ect is concerned.

### EXAMPLES

In the following description, all experiments for which no detailed protocol is given are performed according to standard protocol.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXPERIMENTAL PROCEDURES

### TR1 CELL ISOLATION

Blood samples from healthy patients were collected and white blood cells were separated using gradient density centrifugation. Cells were then cultured in the presence of type II collagen in order to induce the specific proliferation of Tr1 cells directed against this antigen. After 13 days of culture, cell populations were cloned by limiting dilution method. Clones were then assessed for their specificity to type II collagen and for characteristic Tr1 cytokine production profile.

### CYTOKINE ASSAYS

For the determination of antigen specificity, sandwich ELISAs were performed on 48 hours supernatants of T-cell clones stimulated in the presence of antigen presenting cells (4.10⁵) and in the presence or absence of the specific antigen (type II collagen). For the determination of the cytokine production profile, type II collagen Tr1 cell clones were stimulated with anti-CD3 + anti-CD28 monoclonal antibodies and the supernatants were harvested after 48 hours. ELISAs were performed using anti-IL-4 (11B11), anti-IL-10 (2A5), anti-IFN-γ (XGM1.2), biotin anti-IL-4 (24G2), anti-IL-10 (SXC1), anti-IFN-γ (R4-6A2) (Pharmingen Becton Dickinson).

### SUPPRESSION STUDIES

For suppression studies, graded quantities of type II collagen specific Tr1 clones were co-cultured with autologous CD4 positive T lymphocytes. Co-cultures were stimulated with anti-CD3 + anti-CD28 monoclonal antibodies. After 3 days total cell proliferation was assessed using the WST-1 proliferation kit from Roche.

### RESULTS

Figure 1 shows the IL-10 production of two distinct Tr1 cell populations specific for type II collagen in the presence or absence of the antigen. Results show that type II collagen stimulation induces an increase in the production of IL-10. These results demonstrate the specificity of the cell populations toward type II collagen.

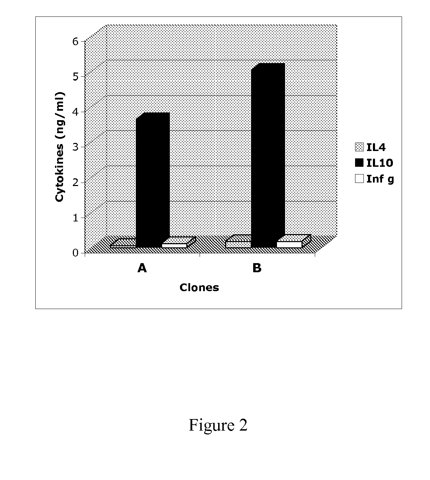

To further determine the cytokine secretion profile of these Tr1 cell populations specific for type II collagen, cells were stimulated in the presence of anti-CD3+anti-CD28 monoclonal antibodies. ELISAs were performed on 48h supernatants to measure IL-4, IL-10 and IFNγ production. Figure 2 shows that the cytokine secretion profile observed for the latter type II collagen specific populations corresponds to a Tr1 cytokine secretion profile, i.e. high production of IL-10, low production of IFNγ and no production of IL-4.

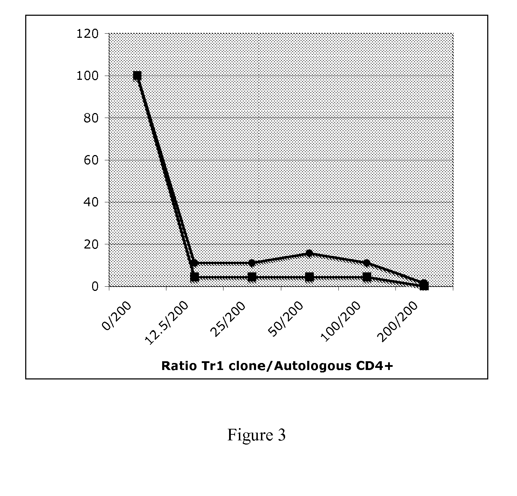

Suppressive activity of these type II collagen Tr1 populations was then assessed. Tr1 cells were co-cultivated with autologous CD4+ T cells in the presence of anti-CD3+anti-CD28 monoclonal antibodies. After 3 days of stimulation, cell proliferation was measured. Figure 3 shows the results for the two Tr1 populations and confirms the suppressive activity of these cells.

## Claims

1. Composition comprising at least one human Tr1 cell population directed against a joint-associated antigen, provided said joint-associated antigen is not HSP, keratin, pyruvate deshydrogenase, topoisomerase I, cardiolipin or collagen type IV.

2. A composition according to claim **1,** wherein said human Tr1 cell population is a human Tr1 clone population.

3. A composition according to anyone of claim **1** or **2,** wherein said joint-associated antigen is selected from the group comprising citrulline-substituted cyclic and linear filaggrin peptides, collagen type II peptides, human cartilage glycoprotein 39 (HCgp39) peptides, heterogenous nuclear ribonucleoprotein (hnRNP) A2 peptides, hnRNP B1, hnRNP D, Ro60/52, BiP, vimentin, fibrinogen, collagen type I, III and V peptides, annexin V, Glucose 6 phosphate isomerase (GPI), acetyl-calpastatin, aldolase, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

4. A composition according to claim **3,** wherein said joint-associated antigen is collagen type II and fragments, variants and mixtures thereof.

5. A composition according to claim **3,** wherein said joint-associated antigen is HCgp39 and fragments, variants and mixtures thereof.

6. Medicament comprising at least one human Tr1 cell population directed against a joint-associated antigen.

7. Pharmaceutical composition comprising at least one human Tr1 cell population directed against a joint-associated antigen in combination with one or more pharmaceutically acceptable carrier.

8. Medicament according to claim **6** or pharmaceutical composition according to claim 7, wherein said human Tr1 cell population is a human Tr1 clone population.

9. Medicament or pharmaceutical composition according to claim **8,** wherein said human Tr1 cell is directed against a joint-associated antigen selected among citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

10. Medicament or pharmaceutical composition according to claim **8,** wherein said human Tr1 cell is directed against a joint-associated antigen selected among collagen type II, HCgp39 and HSP.

11. Use of a composition comprising at least one human Tr1 cell population directed against a joint-associated antigen for the preparation of a medicament or a pharmaceutical composition for treating an arthritic condition.

12. The use according to claim **11,** wherein said joint-associated antigen is selected from the group comprising citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP B1, hnRNP D, Ro60/52, BiP, keratin, vimentin, fibrinogen, cardiolipin, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic phosphatidylserine, neutrally charged phosphatidylethanolamine and phosphatidylcholine, matrix metalloproteinase, fibrillin, aggreccan, and fragments, variants and mixtures thereof.

13. The use according to claim **11,** wherein said at least one human Tr1 cell population is directed against a joint-associated antigen selected among collagen type II, HCgp39 and HSP.

14. The use according to anyone of claim **11** to **13,** wherein said arthritic condition is rheumatoid arthritis.

15. The use according to anyone of claim **11** to **13,** wherein said arthritic condition is ankylosing spondylitis.

16. The use according to anyone of claim **11** to **13,** wherein said arthritic condition is juvenile idiopathic arthritis.

17. The use according to anyone of claim **11** to **13,** wherein said arthritic condition is psoriatic arhritis

18. The use according to anyone of claims **11** to **17,** wherein the medicament or pharmaceutical composition to be administered to a subject in need thereof comprises human Tr1 cells autologous to the cells of said subject.

19. The use according to anyone of claim **11** to **18,** wherein 10⁴/kg to 10⁹/kg Tr1 cells are administered to the subject in need thereof.

20. The use according to anyone of claim **11** to **19,** wherein the administration to said subject of an effective amount of the medicament or the pharmaceutical composition of the invention is in combination with one or more therapeutic agent used for treating an arthritic condition.

21. The use according to claim **20,** wherein the administration to said subject of an effective amount of the medicament or the pharmaceutical composition of the invention is in combination with one or more therapeutic agent selected in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

22. The use according to anyone of claim **11** to **19,** wherein said subject does not respond adequately to, or is unlikely to respond adequately to, one or more therapeutic agent in the group of corticoids, anti-TNF, anti-interleukins, anti-B lymphocytes, anti-costimulatory molecules, tolerogenic agents, anti-complement proteins, inhibitors of T cell signalling molecules, inhibitors of cell migration, methotrexate, leflunomide, sulfasalazine, hydroxychloroquine, azathioprine, methotrexate, cyclosporine, minocycline, D-penicillamine.

23. Process for treating an arthritic condition in a subject in need thereof, said process comprising the steps of:
- obtaining Tr1 cells directed to a selected joint-associated antigen, said Tr1 cells being obtained from a blood sample of said subject,
- cloning said Tr1 cells directed to a selected joint-associated antigen,
- further expanding Tr1 clones obtained at the previous step,
- injecting Tr1 clones thus obtained in said subject, preferably by intravenous route.


SEQUENCE LISTING

<110>  TXCELL
       FOUSSAT, A

<120>  COMPOSITIONS FOR TREATING AN ARTHRITIC CONDITION

<130>  BEP080166

<160>  9

<170>  PatentIn version 3.3

<210>  1
<211>  29
<212>  PRT
<213>  artificial

<220>
<223>  245-273 fragment of type II collagen

<400>  1

Ile Ala Gly Ala Pro Gly Phe Pro Gly Pro Arg Gly Pro Pro Gly Pro
1               5                   10                  15

Gln Gly Ala Thr Gly Pro Leu Gly Pro Lys Gly Gln Thr
            20                  25

<210>  2
<211>  13
<212>  PRT
<213>  artificial

<220>
<223>  HCgp39 epitope

<400>  2

Pro Thr Phe Gly Arg Ser Phe Thr Leu Ala Ser Ser Glu
1               5                   10

<210>  3
<211>  13
<212>  PRT
<213>  artificial

<220>
<223>  HCgp39 epitope

<400>  3

Arg Ser Phe Thr Leu Ala Ser Ser Glu Thr Gly Val Gly
1               5                   10

<210>  4
<211>  13
<212>  PRT
<213>  artificial

<220>
<223>  HCgp39 epitope

<400>  4

Val Gly Tyr Asp Asp Gln Glu Ser Val Lys Ser Lys Val
1               5                   10

<210>  5
<211>  14
<212>  PRT
<213>  artificial

<220>
<223>  HCgp39 epitope

<400>  5

Ser Gln Arg Phe Ser Lys Ile Ala Ser Asn Thr Gln Ser Arg
1               5                   10

<210>  6
<211>  9
<212>  PRT
<213>  artificial

<220>
<223>  HCgp39 epitope

<400>  6

Phe Gly Arg Ser Phe Thr Leu Ala Ser
1               5

<210>  7
<211>  9
<212>  PRT
<213>  artificial

<220>
<223>  HCgp39 epitope

<400>  7

Phe Thr Leu Ala Ser Ser Glu Thr Gly
1               5

<210>  8
<211>  9
<212>  PRT
<213>  artificial

<220>
<223>  HCgp39 epitope

<400>  8

Tyr Asp Asp Gln Glu Ser Val Lys Ser
1               5

<210>  9
<211>  9
<212>  PRT
<213>  artificial

<220>
<223>  HCgp39 epitope

<400>  9

Phe Ser Lys Ile Ala Ser Asn Thr Gln
1               5